# EUROPEAN PATENT APPLICATION

(11) **EP 1 508 610 A1**
(43) Date of publication of application: **23.02.2005**
(21) Application number: 03730728.7
(22) Date of filing: 30.05.2003
(51) Int. Cl.: C10M 133/16, C10M 133/56, C10M 139/00, C10M 141/12

(54) **LUBRICATING OIL ADDITIVE COMPOSITION FOR INTERNAL COMBUSTION ENGINE**

(30) Priority: 30.05.2002 JP 2002156939
(71) Applicant: IDEMITSU KOSAN CO., LTD., Tokyo 100-8321 (JP)
(72) Inventor: KOSHIMA, Hiroaki, Sodegaura-shi, Chiba 299-0205 (JP); TERADA, Izumi, Sodegaura-shi, Chiba 299-0205 (JP); KATAFUCHI, Tadashi, Ichihara-shi, Chiba 299-0107 (JP); KAMANO, Hideki, Ichihara-shi, Chiba 299-0107 (JP)
(74) Representative: Gille Hrabal Struck Neidlein Prop Roos
(86) International application number: PCT/JP2003/006837
(87) International publication number: WO 2003/102117

(57) **Abstract**

The invention provides a lubricating oil additive for internal combustion engines which additive enables to prepare a lubricating oil composition for internal combustion engines having an enhanced base number without impairing the performance of an exhaust gas treatment apparatus such as a PM trap or an oxidation catalyst member; a lubricating oil additive composition for internal combustion engines; and a lubricating oil composition for internal combustion engines containing the lubricating oil additive composition.

The invention is directed to a lubricating oil additive composition for internal combustion engines including a succinimide compound represented by formula (1) : 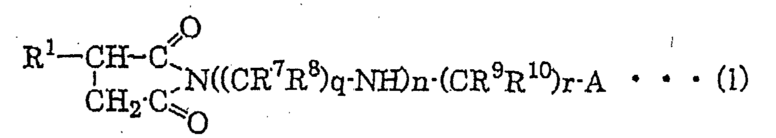 (wherein R¹ represents a C6-C30 alkenyl group or alkyl group; each of R¹, R⁸, R⁹, and R¹⁰ represents hydrogen or a C1-C3 alkyl group; q is an integer from 2 to 4; n is an integer from 0 to 3; r is an integer from 2 to 4; and A represents an amino group or an N-piperazyl group), or a boron-introduced product thereof; and to use thereof.

## Description

### Technical Field

The present invention relates to a lubricating oil additive composition for internal combustion engines, and to a lubricating oil composition for internal combustion engines containing the additive composition. The lubricating oil composition for internal combustion engines exhibits excellent detergency at high temperature, exhibits excellent heat resistance, has low ash content, and does not affect an exhaust gas treatment apparatus such as a particulate matter (hereinafter abbreviated as PM) trap or an oxidation catalyst member for oxidizing uncombusted fuel or lubricating oil, and therefore can meet future emission gas regulations.

### Background Art

Conventionally, lubricating oils for internal combustion engines, particularly those for diesel engines, have employed a metallic detergent-dispersant and an ash-less detergent-dispersant in combination. Generally employed metallic detergent-dispersants include sulfonates, phenates, salicylates, phosphonates of alkali metal or alkaline earth metal, and perbasic compounds thereof.

Environmental pollution caused by nitrogen oxides (hereinafter referred to as NOₓ) and PM contained in exhaust gas from internal combustion engines, *inter alia,* diesel engines, raises a grave problem that remains to be solved, and there is a pressing need to develop effective measures therefor. Therefore, reduction of the NOₓ level and the PM level of exhaust gas is a problem to be immediately solved. NOₓ level has been reduced by taking measures such as efficiently performing exhaust gas recirculation (EGR) and lowering combustion peak temperature by retarding the timing of fuel injection.

However, when the combustion peak temperature is lowered, generation of black smoke and PM increases, thereby requiring provision of an exhaust gas post-treatment apparatus. Such apparatuses which have been studied include a PM trap and an oxidation catalyst member, both of which have a filter-like structure. Thus, when a conventional diesel engine oil is employed, a metallic component contained in the oil causes plugging of the apparatus. When the metallic component level of the oil is lowered (i.e., upon reduction in the amount of a metallic detergent and an anti-wear agent), detergency and wear resistance are deteriorated. In particular, reduction of a metallic detergent leads to a decrease in the initial base number. Thus, there is demand for development of a novel lubricating oil for internal combustion engines in order to maintain long-drain performance (basicity) which is equivalent to that of a conventional engine oil. In other words, a novel lubricating oil additive for internal combustion engines is demanded.

### Disclosure of the Invention

The present invention has been conceived under such circumstances, and an object of the present invention is to provide a lubricating oil additive for internal combustion engines, which additive enables preparation of a lubricating oil composition for internal combustion engines having an enhanced base number without impairing the performance of an exhaust gas treatment apparatus such as a PM trap or an oxidation catalyst member. Another object of the invention is to provide a lubricating oil additive composition for internal combustion engines. Still another object of the invention is to provide a lubricating oil composition for internal combustion engines containing the lubricating oil additive composition.

The present inventors have found that a lubricating oil composition for internal combustion engines having an enhanced base number can be prepared by controlling the molecular weight and the nitrogen content of an alkenyl- or alkylsuccinimide compound or a boron-introduced product thereof serving as a fuel oil additive. The present invention has been accomplished on the basis of this finding.

Accordingly, the gist of the present invention is as follows.
1. A lubricating oil additive composition for internal combustion engines comprising a succinimide compound represented by formula (1): 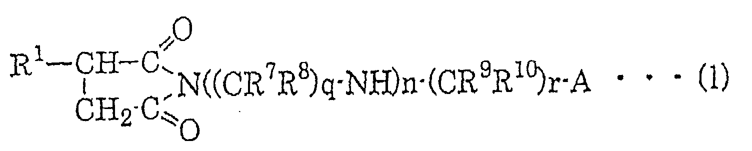 (wherein R¹ represents a C6-C30 alkenyl group or alkyl group; each of R⁷, R⁸, R⁹, and R¹⁰ represents hydrogen or a C1-C3 alkyl group; q is an integer from 2 to 4; n is an integer from 0 to 3, r is an integer from 2 to 4; and A represents an amino group or an N-piperazyl group), or a boron-introduced product thereof.
2. A lubricating oil additive composition for internal combustion engines as described in 1 above, which comprises a succinimide compound represented by formula (1) wherein each of R⁷, R⁸, R⁹, and R¹⁰ represents hydrogen, or a boron-introduced product thereof.
3. A lubricating oil additive composition for internal combustion engines as described in 2 above, which comprises a succinimide compound represented by formula (1) wherein each of q and r is 2, or a boron-introduced product thereof.
4. A lubricating oil additive composition for internal combustion engines as described in 3 above, which comprises a succinimide compound represented by formula (1) wherein R¹ has 10 to 20 carbon atoms, or a boron-introduced product thereof.
5. A lubricating oil additive composition for internal combustion engines comprising a succinimide compound as recited in any of 1 to 4 above, or a boron-introduced product thereof (A), and a substituted hydroxyaromatic ester derivative (B).
6. A lubricating oil additive composition for internal combustion engines as described in 5 above, wherein the substituted hydroxyaromatic ester derivative is represented by formula (2): 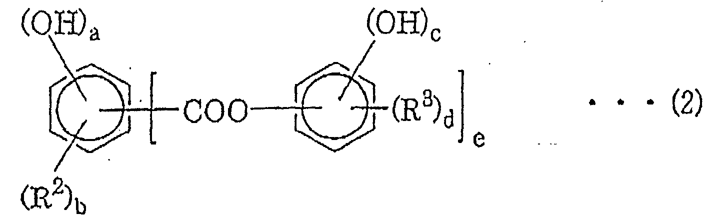 (wherein each of R² and R³, which may be identical to or different from each other, represents a organic group having six or more carbon atoms; a, b, c, d, and e are integers satisfying the relations 1 ≤ a ≤ 3; 1 ≤ b ≤ 3; 0 ≤ c ≤ 3; 1 ≤ d ≤ 3; 1 ≤ e ≤ 3; 3 ≤ (a + b + e) ≤ 6; and 1 ≤ (c + d) ≤ 5; and when a plurality of R²s or R³s are present, these groups may be identical to or different from one another), or is represented by formula (3) : 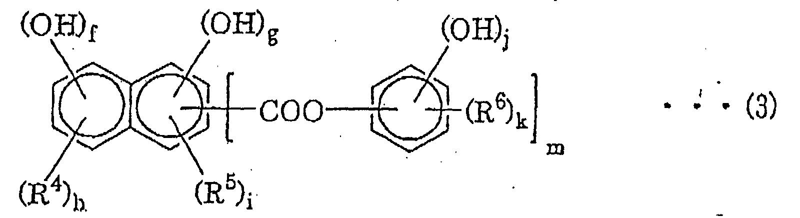 (wherein each of R⁴, R⁵, and R⁶, which may be identical to or different from one another, represents a organic group having six or more carbon atoms; f, g, h, i, j, k, and m are integers satisfying the relations 0 ≤ f ≤ 3; 0 ≤ g ≤ 3; 1 ≤ (f + g) ≤ 3; 0 ≤ h ≤ 4; 0 ≤ i ≤ 3; 1 ≤ (h + i) ≤ 6; 0 ≤ (f + h) ≤ 4; 0 ≤ (g + i + m) ≤ 4; 0 ≤ j ≤ 3; 1 ≤ k ≤ 3; 1 ≤ m ≤ 3; 3 ≤ (f + g + h + i + m) ≤ 8; and 1 ≤ (j + K) ≤ 5; when a plurality of R⁴s, R⁵s, or R⁶s are present, these groups may be identical to or different from one another).
7. A lubricating oil composition for internal combustion engines comprising a lubricating oil additive composition for internal combustion engines as recited in any of 1 to 6 above.

### Best Modes for Carrying Out the Invention

A first aspect of the present invention is drawn to a lubricating oil additive for internal combustion engines, which comprises, a succinimide compound represented by formula (1).

In the aforementioned formula (1), R¹ represents a C6-C30 alkenyl group or alkyl group, which may be a linear chain or a branched chain. When R¹ has five or less carbon atoms, the succinimide compound may fail to be sufficiently dissolved in a medium such as a lubricating base oil, whereas when R¹ has 31 or more carbon atoms, a compound of high base number may fail to be produced. R¹ preferably contains 10 to 20 carbon atoms. Specific examples of the above groups include a decyl group, a decenyl group, a dodecyl group, a dodecenyl group, a tetradecyl group, a tetradecenyl group, a hexadecyl group, a hexadecenyl group, an octadecyl group, an octadecenyl group, an eicosyl group, and an eicosenyl group.

Each of R⁷, R⁸, R⁹, and R¹⁰ represents hydrogen or a C1-C3 alkyl group. When both of R⁷ and R⁸ are hydrogen or either of R⁷ or R⁸ is a methyl group, both of R⁹ and R¹⁰ are preferably hydrogen or either of R⁹ or R¹⁰ is preferably a methyl group. Particularly preferably, all of the R⁷, R⁸, R⁹, and R¹⁰ are hydrogen.

Then, n is an integer from 0 to 3. When n is more than 3, the succinimide compound has excessively large polarity and cannot be sufficiently dissolved in a medium such as a lubricating base oil. The number "n" is adjusted by polyamine serving as a starting material. Examples of the polyamine include alkylenediamines such as ethylenediamine, propanediamine, butanediamine, N-methyl-1,3-propanediamine, and N,N-dimethyl-1,3-propanediamine; polyalkylene-polyamines such as diethylenetriamine, triethylenetetramine, and tetraethylenepentamine; and polyalkylene-polyamines having a cyclic alkyleneamine such as aminoethylpiperazine.

Each of q and r is an integer from 2 to 4.

Although no particular limitation is imposed on the method for producing the aforementioned succinimide compound, the compound is preferably produced through the following method.

Specifically, the compound is produced by reacting an alkenyl- or alkylsuccinic acid or an alkenyl- or alkylsuccinic anhydride (a) with the aforementioned polyamine (b) at a mole ratio of (a) : (b) = 1 : 10 to 10 : 1, preferably 1 : 2 to 2 : 1. The reaction temperature, reaction pressure, and reaction time are preferably 140 to 200°C, 0.1 to 1 MPa(G), and 1 to 10 hours, respectively.

The reaction is not necessarily carried out in the presence of solvent, but a solvent may also be used. The solvent preferably has a boiling point of 140 to 150°C, and examples include toluene and xylene.

When solvent is employed, no particular limitation is imposed on the concentration of a starting material, and the concentration may be elevated to the corresponding saturated solubility. Preferably, the concentration is 0.1 to 10 mol/L.

In a preferred order of addition of components (a) and (b), the component (a) is added to the component (b) during reaction from the viewpoint of suppression of by-products.

The aforementioned boron-introduced product of the succinimide compound of the present invention is obtained by reacting a boron compound (c) with the succinimide compound produced in the above method, the amount of the boron compound being a mole ratio (based on polyamine) 1 : 0.01 to 10, preferably 1 : 0.05 to 5. Examples of the boron compound (c) include boron oxide, boron halides, boric acid, boric anhydride, and boric acid esters. The reaction of the succinimide compound with the compound (c) is performed at about 50 to 250°C, preferably 100 to 200°C. The reaction may be performed in the presence of a solvent such as an organic solvent (e.g., hydrocarbon oil).

The aforementioned succinimide compound or a boron-introduced product thereof has a base number (as determined through HCl method) of 50 mg KOH/g or more, and therefore serves as a detergent-dispersant.

The aforementioned succinimide compound or a boron-introduced product thereof [component (A)] may be employed as one component of the lubricating oil additive composition for internal combustion engines. An example of preferred lubricating oil additive compositions for internal combustion engines contains component (A) with a substituted hydroxyaromatic ester derivative [component (B)]. No particular limitation is imposed on the type of substituted hydroxyaromatic ester derivative, and examples of preferred ester derivatives include compounds represented by formula (2) or (3).

The substituted hydroxyaromatic ester derivative will next be described in detail.

The aforementioned substituted hydroxyaromatic ester derivative is at least one compound selected from substituted hydroxyaromatic carboxylic acid ester derivatives represented by formula (2) or (3). In the above-mentioned formulas (2) and (3), each of R², R³, R⁴, R⁵, and R⁶ represents a organic group having six or more carbon atoms. Examples of the organic group having six or more carbon atoms include hydrocarbon groups having preferably 6 to 100 carbon atoms, more preferably 8 to 20 carbon atoms. Examples of the hydrocarbon groups include an alkyl group, an alkenyl group, a cycloalkyl group, a cycloalkenyl group and an aralkyl group, and these groups may have a non-hydrocarbon substituent and a hetero-atom in a chain or a ring. Specific examples of the hydrocarbon groups include hydrocarbon groups such as a hexyl group, an octyl group, a nonyl group, a decyl group, a dodecyl group, a hexadecyl group and a triacontyl group, and groups derived from olefin polymers such as polyethylene, polypropylene, and polybutene. In the case where the substituted hydroxyaromatic carboxylic acid ester derivative having a low viscosity is desired, it is preferred that R², R³, R⁴, R⁵, and R⁶ are substantially straight-chain hydrocarbon groups. R² and R³ may be identical to or different from each other, and R⁴, R⁵, and R⁶ may be identical to or different from one another.

In the aforementioned formula (2), a, b, c, d, and e are integers satisfying the relations 1 ≤ a ≤ 3; 1 ≤ b ≤ 3; 0 ≤ c ≤ 3; 1 ≤ d ≤ 3; 1 ≤ e ≤ 3; 3 ≤ (a + b + e) ≤ 6; and 1 ≤ (c + d) ≤ 5. In the case where b is 2 or 3, a plurality of R²s may be identical to or different from one another, and in the case where d is 2 or 3, a plurality of R³s may be identical to or different from one another.

In the aforementioned formula (3), f, g, h, i, j, k, and m are integers satisfying the relations 0 ≤ f ≤ 3; 0 ≤ g ≤ 3; 1 ≤ (f + g) ≤ 3; 0 ≤ h ≤ 4; 0 ≤ i ≤ 3; 1 ≤ (h + i) ≤ 6; 0 ≤ (f + h) ≤ 4; 0 ≤ (g + i + m) ≤ 4; 0 ≤ j ≤ 3; 1 ≤ k ≤ 3; 1 ≤ m ≤ 3; 3 ≤ (f + g + h + i + m) ≤ 8; and 1 ≤ (j + K) ≤ 5. In the case where h is 2, 3, or 4, a plurality of R⁴ s may be identical to or different from one another, and in the case where i is 2 or 3, a plurality of R⁵s may be identical to or different from one another. Moreover, in the case where k is 2 or 3, a plurality of R⁶s may be identical to or different from one another.

Specific examples of the substituted hydroxyaromatic carboxylic acid ester derivatives represented by formula (2) include (hexylhydroxybenzoic acid) hexylphenyl ester, (hexylhydroxybenzoic acid) dodecylphenyl ester, (octylhydroxybenzoic acid) octylphenyl ester, (nonylhydroxybenzoic acid) nonylphenyl ester, (nonylhydroxybenzoic acid) hexadecylphenyl ester, (dodecylhydroxybenzoic acid) nonylphenyl ester, (dodecylhydroxybenzoic acid) dodecylphenyl ester, (dodecylhydroxybenzoic acid) hexadecylphenyl ester, (hexadecylhydroxybenzoic acid) hexylphenyl ester, (hexadecylhydroxybenzoic acid) dodecylphenyl ester, (hexadecylhydroxybenzoic acid) hexadecylphenyl ester, eicosylhydroxybenzoic acid) eicosylphenyl ester, (mixed C11-C15 alkylhydroxybenzoic acid) mixed C11-C15 alkylphenyl esters, long-chain alkyl (e.g., a group derived from a polydecene having 30 or more carbon atoms, or a group derived from a polybutene having a weight-average molecular weight of 400 or more) hydroxybenzoic acid dodecylphenyl ester, long-chain alkyl (e.g., a group derived from a polydecene having 30 or more carbon atoms, or a group derived from a polybutene having a weight-average molecular weight of 400 or more) hydroxybenzoic acid long-chain alkyl (e.g., a group derived from a polydecene having 30 or more carbon atoms, or a group derived from a polybutene having a weight-average molecular weight of 400 or more) phenyl ester, (hexylhydroxybenzoic acid) hexylhydroxyphenyl ester, (octylhydroxybenzoic acid) octylhydroxyphenyl ester, (dodecylhydroxybenzoic acid) nonylhydroxyphenyl ester, (dodecylhydroxybenzoic acid) dodecylhydroxyphenyl ester, (hexadecylhydroxybenzoic acid) doecylhydroxyphenyl ester, (hexadecylhydroxybenzoic acid) hexadecylhydroxyphenyl ester, (eicosylhydroxybenzoic acid) eicosylhydroxyphenyl ester, (mixed C11-C15 alkylhydroxybenzoic acid) mixed C11-C15 alkylhydroxyphenyl esters, long-chain alkyl (e.g., a group derived from a polydecene having 30 or more carbon atoms, or a group derived from a polybutene having a weight-average molecular weight of 400 or more) hydroxybenzoic acid dodecylhydroxyphenyl ester, long-chain alkyl (e.g., a group derived from a polydecene having 30 or more carbon atoms, or a group derived from a polybutene having a weight-average molecular weight of 400 or more) hydroxybenzoic acid long-chain alkyl (e.g., a group derived from a polydecene having 30 or more carbon atoms, or a group derived from a polybutene having a weight-average molecular weight of 400 or more) hydroxyphenyl ester, (hexyldihydroxybenzoic acid) hexylphenyl ester, (nonyldihydroxybenzoic acid) nonylphenyl ester, (nonyldihydroxybenzoic acid) dodecylphenyl ester, (dodecyldihydroxybenzoic acid) nonylphenyl ester, (dodecyldihydroxybenzoic acid) dodecylphenyl ester, (hexadecyldihydroxybenzoic acid) hexadecylphenyl ester, (eicosyldihydroxybenzoic acid) hexadecylphenyl ester, (eicosyldihydroxybenzoic acid) eicosylphenyl ester, (mixed C11-C15 alkyldihydroxybenzoic acid) mixed C11-C15 alkylphenyl esters, long-chain alkyl (e.g., a group derived from a polydecene having 30 or more carbon atoms, or a group derived from a polybutene having a weight-average molecular weight of 400 or more) dihydroxybenzoic acid dodecylphenyl ester, long-chain alkyl (e.g., a group derived from a polydecene having 30 or more carbon atoms, or a group derived from a polybutene having a weight-average molecular weight of 400 or more) dihydroxybenzoic acid long-chain alkyl (e.g., a group derived from a polydecene having 30 or more carbon atom, or a group derived from a polybutene having a weight-average molecular weight of 400 or more) phenyl ester, (hexyldihydroxybenzoic acid) hexylhydroxyphenyl ester, (nonyldihydroxybenzoic acid) nonylhydroxyphenyl ester, (nonyldihydroxybenzoic acid) dodecylhydroxyphenyl ester, (dodecyldihydroxybenzoic acid) nonylhydroxyphenyl ester, (dodecyldihydroxybenzoic acid) dodecylhydroxyphenyl ester, (hexadecyldihydroxybenzoic acid) hexadecylhydroxyphenyl ester, (eicosyldihydroxybenzoic acid) hexadecylhydroxyphenyl ester, (eicosyldihydroxybenzoic acid) eicosylhydroxyphenyl ester, (mixed C11-C15 alkyldihydroxybenzoic acid) mixed C11-C15 alkylhydroxyphenyl esters, long-chain alkyl (e.g., a group derived from a polydecene having 30 or more carbon atoms, or a group derived from a polybutene having a weight-average molecular weight of 400 or more) dihydroxybenzoic acid dodecylhydroxyphenyl ester, and long-chain alkyl (e.g., a group derived from a polydecene having 30 or more carbon atoms, or a group derived from a polybutene having a weight-average molecular weight of 400 or more) dihydroxybenzoic acid long-chain alkyl (e.g., a group derived from a polydecene having 30 or more carbon atoms, or a group derived from a polybutene having a weight-average molecular weight of 400 or more) hydroxyphenyl ester.

Specific examples of the substituted hydroxyaromatic carboxylic acid ester derivatives represented by formula (3) include (hexylhydroxynaphthoic acid) hexylphenyl ester, (hexylhydroxynaphthoic acid) hexadecylphenyl ester, (nonylhydroxynaphthoic acid) nonylphenyl ester, (dodecylhydroxynaphthoic acid) dodecylphenyl ester, (hexadecylhydroxynaphthoic acid) hexadecylphenyl ester, (dodecylhydroxynaphthoic acid) eicosylphenyl ester, (eicosylhydroxynaphthoic acid) eicosylphenyl ester, (mixed C11-C15 alkylhydroxynaphthoic acid) mixed C11-C15 alkylphenyl esters, long-chain alkyl (e.g., a group derived from a polydecene having 30 or more carbon atoms, or a group derived from a polybutene having a weight-average molecular weight of 400 or more) hydroxynaphthoic acid dodecylphenyl ester, long-chain alkyl (e.g., a group derived from a polydecene having 30 or more carbon atoms, or a group derived from a polybutene having a weight-average molecular weight of 400 or more) hydroxynaphthoic acid long-chain alkyl (e.g., a group derived from a polydecene having 30 or more carbon atoms, or a group derived from a polybutene having a weight-average molecular weight of 400 or more) phenyl ester, (hexylhydroxynaphthoic acid) dodecylhydroxyphenyl ester, (octylhydroxynaphthoic acid) dodecylhydroxyphenyl ester, (dodecylhydroxynaphthoic acid) dodecylhydroxyphenyl ester, (dodecylhydroxynaphthoic acid) hexadecylhydroxyphenyl ester, (hexadecylhydroxynaphthoic acid) hexadecylhydroxyphenyl ester, (hexadecylhydroxynaphthoic acid) eicosylhydroxyphenyl ester, (mixed C11-C15 alkylhydroxynaphthoic acid) mixed C11-C15 alkylhydroxyphenyl esters, long-chain alkyl (e.g., a group derived from a polydecene having 30 or more carbon atoms, or a group derived from a polybutene having a weight-average molecular weight of 400 or more) hydroxynaphthoic acid dodecylhydroxyphenyl ester, long-chain alkyl (e.g., a group derived from a polydecene having 30 or more carbon atoms, or a group derived from a polybutene having a weight-average molecular weight of 400 or more) hydroxynaphthoic acid long-chain alkyl (e.g., a group derived from a polydecene having 30 or more carbon atoms, or a group derived from a polybutene having a weight-average molecular weight of 400 or more) hydroxyphenyl ester, (hexyldihydroxynaphthoic acid) hexylphenyl ester, (hexyldihydroxynaphthoic acid) hexadecylphenyl ester, (nonyldihydroxynaphthoic acid) nonylphenyl ester, (dodecyldihydroxynaphthoic acid) dodecylphenyl ester, (dodecyldihydroxynaphthoic acid) eicosylphenyl ester, (hexadecyldihydroxynaphthoic acid) hexadecylphenyl ester, (eicosyldihydroxynaphthoic acid) eicosylphenyl ester, (mixed C11-C15 alkyldihydroxynaphthoic acid) mixed C11-C15 alkylphenyl esters, long-chain alkyl (e.g., a group derived from a polydecene having 30 or more carbon atoms, or a group derived from a polybutene having a weight-average molecular weight of 400 or more) dihydroxynaphthoic acid dodecylphenyl ester, long-chain alkyl (e.g., a group derived from a polydecene having 30 or more carbon atoms, or a group derived from a polybutene having a weight-average molecular weight of 400 or more) dihydroxynaphthoic acid long-chain alkyl (e.g., a group derived from a polydecene having 30 or more carbon atoms, or a group derived from a polybutene having a weight-average molecular weight of 400 or more) phenyl ester, (hexyldihydroxynaphthoic acid) dodecylhydroxyphenyl ester, (octyldihydroxynaphthoic acid) dodecylhydroxyphenyl ester, (dodecyldihydroxynaphthoic acid) dodecylhydroxyphenyl ester, (dodecyldihydroxynaphthoic acid) hexadecylhydroxyphenyl ester, (hexadecyldihydroxynaphthoic acid) hexadecylhydroxyphenyl ester, (hexadecyldihydroxynaphthoic acid) eicosylhydroxyphenyl ester, (mixed C11-C15 alkyldihydroxynaphthoic acid) mixed C11-C15 alkylhydroxyphenyl esters, long-chain alkyl (e.g., a group derived from a polydecene having 30 or more carbon atoms, or a group derived from a polybutene having a weight-average molecular weight of 400 or more) dihydroxynaphthoic acid dodecylhydroxyphenyl ester, and long-chain alkyl (e.g., a group derived from a polydecene having 30 or more carbon atoms, or a group derived from a polybutene having a weight-average molecular weight of 400 or more) dihydroxynaphthoic acid long-chain alkyl (e.g., a group derived from a polydecene having 30 or more carbon atoms, or a group derived from a polybutene having a weight-average molecular weight of 400 or more) hydroxyphenyl ester.

Of these substituted hydroxyaromatic carboxylic acid ester derivatives, more preferable examples are compounds represented by formula (4) : 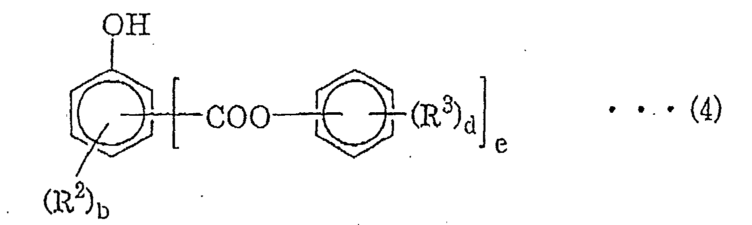 (wherein R², R³, b, d, and e are as defined above, and the sum of b and e falls within a range of 2 to 5), and compounds represented by formula (5): 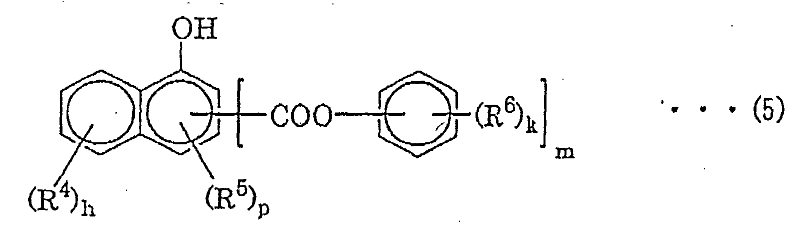 (wherein R⁴, R⁵, R⁶, h, k, and m are as defined above, p is 0, 1 or 2, the sum of h and p falls within a range of 1 to 6, and the sum of h, p, and m falls within a range of 2 to 7).

In the lubricating oil additive composition of the present invention for internal combustion engines, component (B) may be composed of a single compound or two or more compounds represented by the aforementioned formula (2), or may be composed of a single compound or two or more compounds represented by the aforementioned formula (3). Alternatively, component (B) may be composed of one or more compounds represented by formula (2) and one or more compounds represented by formula (3).

In the aforementioned lubricating oil additive composition for internal combustion engines, the proportion by mass of component (A) to component (B) falls within a range of 1 : 99 to 99 : 1, preferably 10 : 90 to 90 : 10.

The lubricating oil composition for internal combustion engines may be prepared by incorporating the lubricating oil additive composition(s) for internal combustion engines to mineral oil or synthetic oil serving as a lubricating base oil in an amount of 0.1 to 50 mass%. The amount of the additives incorporated into the lubricating oil composition preferably falls within a range of 0.1 to 30 mass%.

No particular limitation is imposed on the type of the mineral oil and synthetic oil serving as a base oil for lubricating oil, and any oils generally employed as base oils in lubricating oil for internal combustion engines may be used. However, the base oil preferably has a kinematic viscosity at 100°C of 2 to 35 mm²/s, more preferably 3 to 25 mm²/s. When the kinematic viscosity of the base oil is higher than 35 mm²/s, the fuel cost may undesirably increase, whereas when the kinematic viscosity lower than 2 mm²/s, the lubricating oil may have poor lubricating performance and high volatility may cause excessive oil consumption, which is not preferred. Although no particular limitation is imposed on the pour point of the base oil, serving as an index for low-temperature flowability of the base oil, the pour point is preferably -10°C or lower in general.

These mineral oils and synthetic oils may be selected appropriately from a variety of species. Examples of the mineral oil include paraffin-base mineral oils, naphthenic mineral oils, and their mixed base oils. More specific examples include solvent-purified or hydrogenated, light neutral oils, medium-gravity neutral oils, heavy neutral oils, and bright stocks.

Examples of the synthetic oil include poly-α-olefins, α-olefin copolymers, polybutenes, alkylbenzenes, polyolesters, esters of dibasic acids, esters of polyhydric alcohols, polyoxyalkylene glycols, polyoxyalkylene glycol esters, and polyoxyalkylene glycol ethers. These base oils may be used singly or in combination of two or more species. Mineral oil and synthetic oil may be used in combination.

So long as the effect of the succinimide compound is not impaired, the lubricating oil composition of the present invention for internal combustion engines may further contain additives generally incorporated into lubricating oil such as an anti-oxidant, an anti-wear agent, other detergent-dispersants, a viscosity index improver, and a pour point improver.

### Examples

The present invention will next be described in more detail by way of examples, which should not be construed as limiting the invention thereto.

### [Example 1]

Diethylenetriamine (DETA) (10.3 g, 0.1 mol) dissolved in xylene (20 mL) was placed in a 200-mL separable flask, and purged with nitrogen. The temperature of the solution was maintained at 50°C. To the solution, hexadecenylsuccinic anhydride (HDSA) (32.4 g, 0.1 mol) dissolved in xylene (50 mL) and a mineral oil (17.5 g, equivalent to 150 neutral and to about 30 mass% in the final product) were added dropwise under sufficient stirring conditions. The temperature of the reaction mixture rose to about 80°C through exothermic reaction heat. The mixture was allowed to react at about 100°C for four hours under nitrogen flow while dehydration was performed. Thereafter, unreacted DETA, formed water, and xylene were removed under reduced pressure, followed by cooling and filtration, thereby yielding 57 g of hexadecenylsuccinimide having a base number (as determined through the HCl method) of 101 mg KOH/g.

### [Example 2]

The procedure of Example 1 was repeated, except that aminoethylpiperazine (AEP) (12.9 g, 0.1 mol) was used instead of DETA, to thereby yield 58 g of hexadecenylsuccinimide having a base number of 98 mg KOH/g.

### [Example 3]

The procedure of Example 1 was repeated, except that a mixture of AEP (6.5 g, 0.05 mol) and DETA (5.2 g, 0.05 mol) was used instead of DETA, to thereby yield 57 g of hexadecenylsuccinimide having a base number of 93 mg KOH/g.

### [Example 4]

A mixture of AEP (6.5 g, 0.05 mol) and DETA (5.2 g, 0.05 mol) dissolved in xylene (20 mL) was placed in a 200-mL separable flask, and purged with nitrogen. The temperature of the solution was maintained at 50°C. To the solution, dodecenylsuccinic anhydride (DDSA) (26.7 g, 0.1 mol) dissolved in xylene (50 mL) and a mineral oil (15.5 g, equivalent to 150 neutral and to about 30 mass% in the final product) were added dropwise under sufficient stirring conditions. The mixture was allowed to react at about 150°C for four hours under nitrogen flow while dehydration was performed. Thereafter, unreacted AEP, unreacted DETA, formed water, and xylene were removed under reduced pressure, followed by cooling and filtration, thereby yielding 50 g of dodecenylsuccinimide having a base number of 129 mg KOH/g.

### [Example 5]

The procedure of Example 4 was repeated, except that octadecenylsuccinic anhydride (ODSA) (35.2 g, 0.1 mol) and a mineral oil (18.5 g, equivalent to 150 neutral and to about 30 mass% in the final product) were used instead of DDSA, to thereby yield 59 g of octadecenylsuccinimide having a base number of 89 mg KOH/g.

### [Example 6]

The procedure of Example 1 was repeated, except that 1,3-propanediamine (PDA) (7.4 g, 0.1 mol) was used instead of DETA, to thereby yield 54 g of hexadecenylsuccinimide having a base number of 65 mg KOH/g.

### [Example 7]

The procedure of Example 1 was repeated, except that N-methyl-1,3-propanediamine (MePDA) (8.8 g, 0.1 mol) was used instead of DETA, to thereby yield 54 g of hexadecenylsuccinimide having a base number of 66 mg KOH/g.

### [Example 8]

The procedure of Example 1 was repeated, except that N,N-dimethyl-1,3-propanediamine (diMePDA) (10.2 g, 0.1 mol) was used instead of DETA, to thereby yield 56 g of hexadecenylsuccinimide having a base number of 95 mg KOH/g.

Properties of products obtained in Examples 1 to 8 are shown in Table 1.

### [Examples 9 to 13]

Each (50 g) of the imide compounds produced in Examples 1 to 5 and boric acid (5.8 g) were placed in a 200-mL separable flask, and the mixture was allowed to react at about 150°C for four hours under nitrogen flow. Formed water was removed at 150°C under reduced pressure, followed by filtration, thereby yielding a product. Properties of the products are shown in Table 1.

### [Examples 14 to 16]

Each (50 g) of the imide compounds produced in Examples 6 to 8 and boric acid (1.4 g) were placed in a 200-mL separable flask, and the mixture was allowed to react at about 150°C for four hours under nitrogen flow. Formed water was removed at 150°C under reduced pressure, followed by filtration, thereby yielding a product. Properties of the products are shown in Table 1.

### [Examples 17 to 24]

Each of the succinimide compounds obtained in Examples 1 to 8 was incorporated at 10 mass% into a mineral oil (500-neutral fraction), to thereby prepare a lubricating oil composition. The thus-obtained lubricating oil compositions were evaluated in terms of performance thereof on the basis of a hot tube test. The results are shown in Table 2.

### [Examples 25 to 32]

(Dodecylsalicylic acid) phenyl ester (2 mass%) and each (10 mass%) of the succinimide compounds obtained in Examples 1 to 8 were incorporated into a mineral oil (500-neutral fraction), to thereby prepare a lubricating oil composition. The thus-obtained lubricating oil compositions were evaluated in terms of performance thereof on the basis of a hot tube test. The results are shown in Table 3.

### [Examples 33 to 40]

Each (10 mass%) of the boron-introduced products of the succinimide compounds obtained in Examples 9 to 16 was incorporated into a mineral oil (500-neutral fraction), to thereby prepare a lubricating oil composition. The thus-obtained lubricating oil compositions were evaluated in terms of performance thereof on the basis of a hot tube test. The results are shown in Table 4.

### [Examples 41 to 48]

(Dodecylsalicylic acid) dodecylphenyl ester (2 mass%) and each (10 mass%) of the boron-introduced products of the succinimide compounds obtained in Examples 9 to 16 were incorporated into a mineral oil (500-neutral fraction), to thereby prepare a lubricating oil composition. The thus-obtained lubricating oil compositions were evaluated in terms of performance thereof on the basis of a hot tube test. The results are shown in Table 5.

### [Comparative Example 1]

Polybutene (Mn: 987) (1,100 g), cetyl bromide (6.4 g, 0.021 mol), and maleic anhydride (115 g, 1.2 mol) were placed in a 1-L autoclave, and the mixture was purged with nitrogen. Subsequently, the mixture was allowed to react at 240°C for five hours, followed by cooling to 215°C. Unreacted maleic anhydride and unreacted cetyl bromide were removed from the reaction mixture under reduced pressure, followed by cooling to 140°C and filtration, to thereby yield 1,100 g of polybutenylsuccinic anhydride having a saponification number of 80 mg KOH/g. The thus-obtained polybutenylsuccinic anhydride (100 g), triethylenetetramine (TETA) (9.9 g, 0.068 mol), and a mineral oil (50 g) were placed in a 500-mL separable flask, and the mixture was allowed to react at 150°C for two hours under nitrogen flow. The reaction mixture was heated to 200°C, and unreacted TETA and formed water were removed under reduced pressure, followed by cooling to 140°C and filtration, to thereby yield 153 g of polybutenylsuccinimide having a base number of 44 mg KOH/g.

### [Comparative Example 2]

The procedure of Comparative Example 1 was repeated, except that diethylenetriamine (DETA) (7 g, 0.068 mol) was used instead of DETA, to thereby yield 151 g of polybutenylsuccinimide having a base number of 33 mg KOH/g.

### [Comparative Example 3]

The procedure of Comparative Example 1 was repeated, except that aminoethylpiperazine (AEP) (8.8 g, 0.068 mol) was used instead of DETA, to thereby yield 151 g of polybutenylsuccinimide having a base number of 31 mg KOH/g.

### [Comparative Example 4]

Polybutenylsuccinimide produced in Comparative Example 1 (50 g) and boric acid (5.8 g) were placed in a 200-mL separable flask, and the mixture was allowed to react at about 150°C for four hours under nitrogen flow. Formed water was removed at 150°C under reduced pressure, followed by filtration.

Properties of the products obtained in Comparative Examples 1 to 4 are shown in Table 1.

### [Comparative Examples 5 to 7]

Each of the polybutenylsuccinimide products obtained in Comparative Examples 1 to 3 was incorporated at 10 mass% into a mineral oil (500-neutral fraction), to thereby prepare a lubricating oil composition. The thus-obtained lubricating oil compositions were evaluated in terms of performance thereof on the basis of a hot tube test. The results are shown in Table 2.

### [Comparative Example 8]

Polybutenylsuccinimide obtained in Comparative Example 4 was incorporated at 10 mass% into a mineral oil (500-neutral fraction), to thereby prepare a lubricating oil composition. The thus-obtained lubricating oil composition was evaluated in terms of performance thereof on the basis of a hot tube test. The results are shown in Table 4.

The hot tube test was carried out in the following manner.

A sample oil and air were caused to continuously flow at 0.3 mL/hr and 10 mL/min, respectively, through a glass tube having an inner diameter of 2 mm for 16 hours, while the temperature of the glass tube was maintained at a temperature predetermined for evaluation. Next, lacquer deposited onto the glass tube was compared to a color specimen, and was graded on the basis of standards of transparency=10 points and black=0 point. The higher the grade, the more excellent the performance of the sample oil (i.e., the higher the residual base number of the evaluated oil).

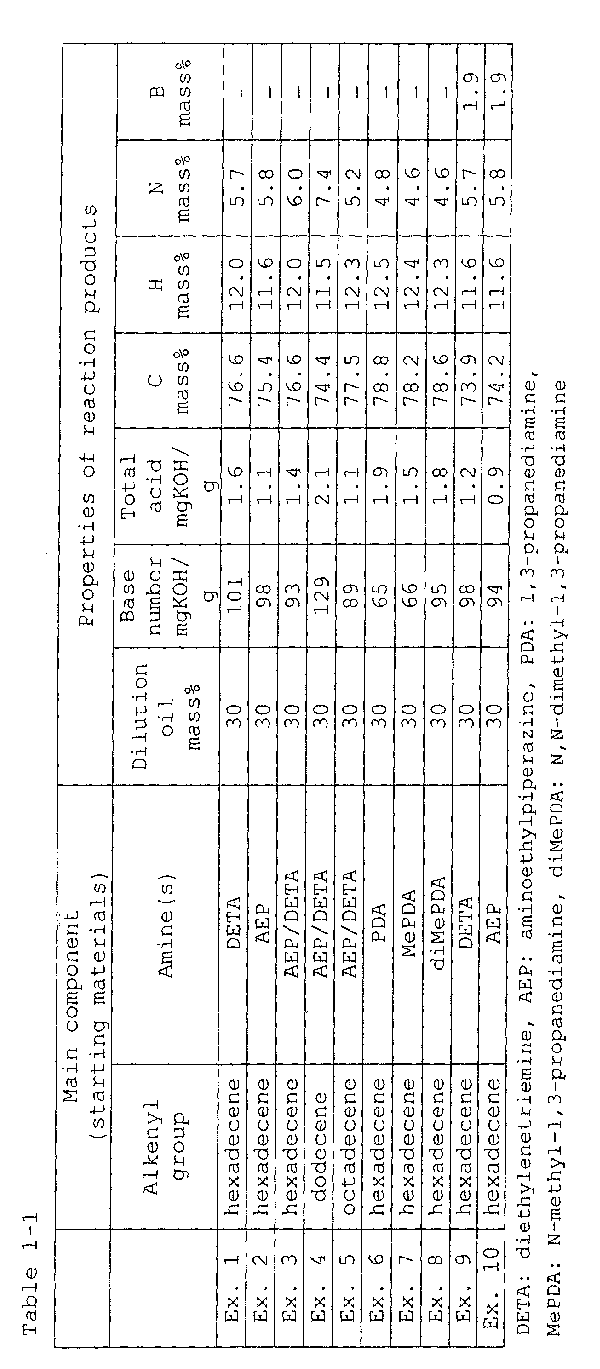

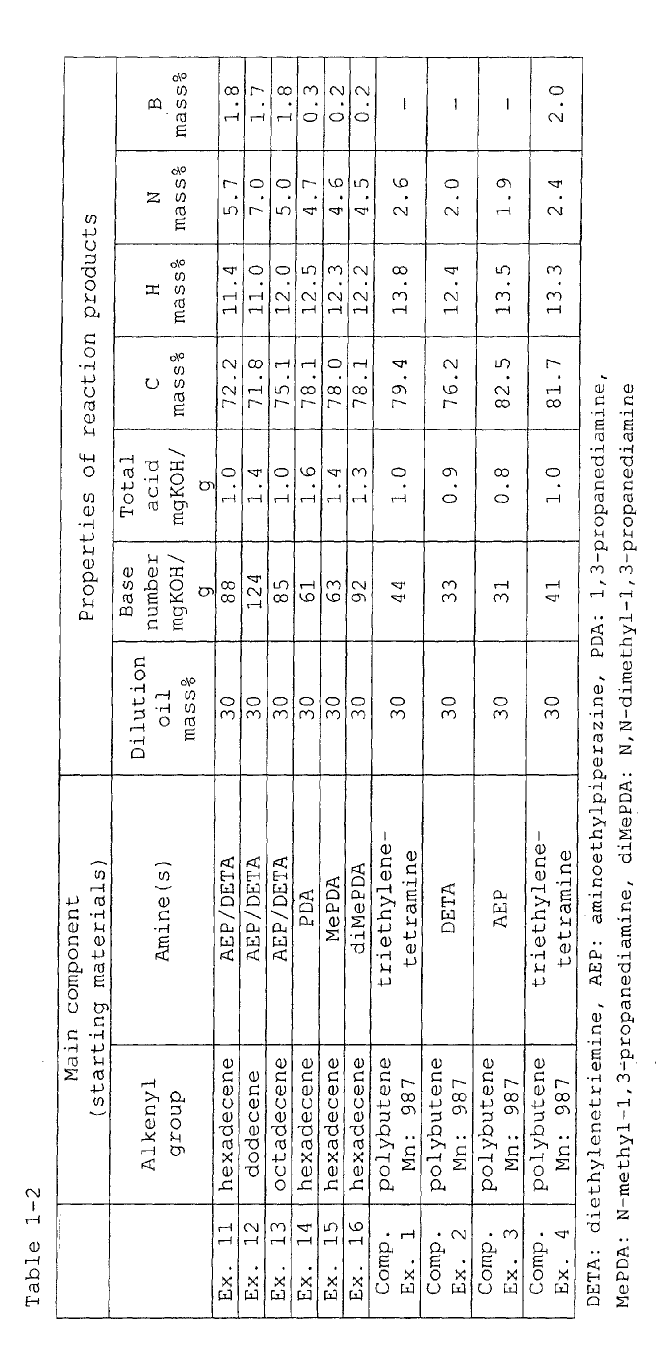

**Table 2**

| | Imide | Hot tube test (230°C) | |
|---|---|---|---|
| | | Grade | Residual base number (mgKOH/g) |
| Ex. 17 | Ex. 1 | 5 | 3.6 |
| Ex. 18 | Ex. 2 | 5 | 3.8 |
| Ex. 19 | Ex. 3 | 5 | 3.8 |
| Ex. 20 | Ex. 4 | 5 | 4.2 |
| Ex. 21 | Ex. 5 | 5 | 3.5 |
| Ex. 22 | Ex. 6 | 4 | 1.5 |
| Ex. 23 | Ex. 7 | 4 | 1.8 |
| Ex. 24 | Ex. 8 | 4 | 2.1 |
| Comp. Ex. 5 | Comp. Ex. 1 | 1 | 0.1 |
| Comp. Ex. 6 | Comp. Ex. 2 | 1 | 0.1 |
| Comp. Ex. 7 | Comp. Ex. 3 | 1 | 0.1 |

**Table 3**

| | Imide | Hot tube test (230°C) | |
|---|---|---|---|
| | | Grade | Residual base number (mgKOH/g) |
| Ex. 25 | Ex. 1 | 6 | 4.5 |
| Ex. 26 | Ex. 2 | 6 | 5.1 |
| Ex. 27 | Ex. 3 | 6 | 4.8 |
| Ex. 28 | Ex. 4 | 6 | 6.1 |
| Ex. 29 | Ex. 5 | 7 | 4.5 |
| Ex. 30 | Ex. 6 | 7 | 4.9 |
| Ex. 31 | Ex. 7 | 7 | 5.3 |
| Ex. 32 | Ex. 8 | 7 | 5.8 |

**Table 4**

| | Imide | Hot tube test (250°C) | |
|---|---|---|---|
| | | Grade | Residual base number (mgKOH/g) |
| Ex. 33 | Ex. 9 | 8 | 6.3 |
| Ex. 34 | Ex. 10 | 8 | 6.5 |
| Ex. 35 | Ex. 11 | 8 | 6.6 |
| Ex. 36 | Ex. 12 | 8 | 8.0 |
| Ex. 37 | Ex. 13 | 8 | 6.2 |
| Ex. 38 | Ex. 14 | 6 | 2.8 |
| Ex. 39 | Ex. 15 | 6 | 2.7 |
| Ex. 40 | Ex. 16 | 6 | 3.4 |
| Comp. Ex. 8 | Comp. Ex. 4 | 2 | 0.3 |

**Table 5**

| | Imide | Hot tube test (270°C) | |
|---|---|---|---|
| | | Grade | Residual base number (mgKOH/g) |
| Ex. 41 | Ex. 9 | 9 | 3.6 |
| Ex. 42 | Ex. 10 | 9 | 4.1 |
| Ex. 43 | Ex. 11 | 9 | 3.8 |
| Ex. 44 | Ex. 12 | 9 | 4.4 |
| Ex. 45 | Ex. 13 | 9 | 3.6 |
| Ex. 46 | Ex. 14 | 7 | 1.7 |
| Ex. 47 | Ex. 15 | 7 | 1.7 |
| Ex. 48 | Ex. 16 | 7 | 2.5 |

### Industrial Applicability

According to the present invention, there can be provided a lubricating oil additive for internal combustion engines which additive enables to prepare a lubricating oil composition for internal combustion engines having an enhanced base number without impairing the performance of an exhaust gas treatment apparatus such as a PM trap or an oxidation catalyst member. In addition, a lubricating oil additive composition for internal combustion engines and a lubricating oil composition for internal combustion engines containing the lubricating oil additive composition can be also provided.

## Claims

1. A lubricating oil additive composition for internal combustion engines comprising a succinimide compound represented by formula (1): 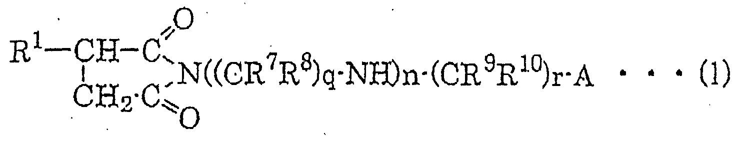 (wherein R¹ represents a C6-C30 alkenyl group or alkyl group; each of R⁷, R⁸, R⁹, and R¹⁰ represents hydrogen or a C1-C3 alkyl group; q is an integer from 2 to 4; n is an integer from 0 to 3; r is an integer from 2 to 4; and A represents an amino group or an N-piperazyl group), or a boron-introduced product thereof.

2. A lubricating oil additive composition for internal combustion engines as described in claim 1, which comprises a succinimide compound represented by formula (1) wherein each of R⁷, R⁸, R⁹, and R¹⁰ represents hydrogen, or a boron-introduced product thereof.

3. A lubricating oil additive composition for internal combustion engines as described in claim 2, which comprises a succinimide compound represented by formula (1) wherein each of q and r is 2, or a boron-introduced product thereof.

4. A lubricating oil additive composition for internal combustion engines as described in claim 3, which comprises a succinimide compound represented by formula (1) wherein R¹ has 10 to 20 carbon atoms, or a boron-introduced product thereof.

5. A lubricating oil additive composition for internal combustion engines comprising a succinimide compound as recited in any of claims 1 to 4, or a boron-introduced product thereof (A) and a substituted hydroxyaromatic ester derivative (B).

6. A lubricating oil additive composition for internal combustion engines as described in claim 5, wherein the substituted hydroxyaromatic ester derivative is represented by formula (2) : 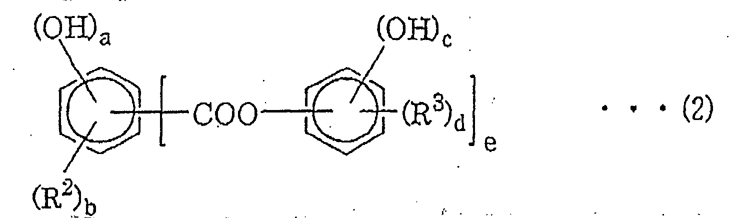 (wherein each of R² and R³, which may be identical to or different from each other, represents a organic group having six or more carbon atoms; a, b, c, d, and e are integers satisfying the relations 1 ≤ a ≤ 3; 1 ≤ b ≤ 3; 0 ≤ c ≤ 3; 1 ≤ d ≤ 3; 1 ≤ e ≤ 3; 3 ≤ (a + b + e) ≤ 6; and 1 ≤ (c + d) ≤ 5; and when a plurality of R²s or R³s are present, these groups may be identical to or different from one another) or is represented by formula (3): 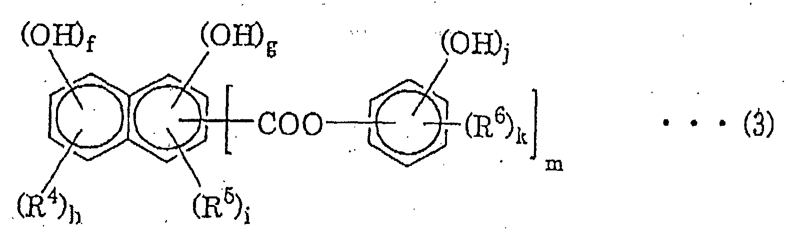 (wherein each of R⁴, R⁵, and R⁶, which may be identical to or different from one another, represents a organic group having six or more carbon atoms; f, g, h, i, j, k, and m are integers satisfying the relations 0 ≤ f ≤ 3; 0 ≤ g ≤ 3; 1 ≤ (f + g) ≤ 3; 0 ≤ h ≤ 4; 0 ≤ i ≤ 3; 1 ≤ (h + i) ≤ 6; 0 ≤ (f + h) ≤ 4; 0 ≤ (g + i + m) ≤ 4; 0 ≤ j ≤ 3; 1 ≤ k ≤ 3; 1 ≤ m ≤ 3; 3 ≤ (f + g + h + i + m) ≤ 8; and 1 ≤ (j + K) ≤ 5; when a plurality of R⁴s, R⁵s, or R⁶s are present, these groups may be identical to or different from one another).

7. A lubricating oil composition for internal combustion engines comprising a lubricating oil additive composition for internal combustion engines as recited in any of claims 1 to 6.
